(19) Europäisches Patentamt
European Patent Office
Office européen des brevets

(11) Veröffentlichungsnummer: **0 216 378**
**A2**

(12) **EUROPÄISCHE PATENTANMELDUNG**

(21) Anmeldenummer: 86113182.9

(51) Int. Cl.⁴: **A61L 15/04**

(22) Anmeldetag: 25.09.86

(30) Priorität: 25.09.85 CS 6840/85

(43) Veröffentlichungstag der Anmeldung:
01.04.87 Patentblatt 87/14

(84) Benannte Vertragsstaaten:
AT CH DE FR GB IT LI NL SE

(71) Anmelder: **Státni vyzkumny ustav textilni**
**U Jezu 2**
**Liberec(CS)**

(72) Erfinder: **Mozisek, Maxmilian, RNDr CSc**
**Prokofevova 2**
**Brno(CS)**
Erfinder: **Cerny, Pavel, RNDr DrSc**
**Divadelni 3**
**Brno(CS)**
Erfinder: **Mozisková, Jarmila**
**Prokofevova 2**
**Brno(CS)**
Erfinder: **Smekal, Miroslav, MUDr CSc**
**Jindrichova 6**
**Brno(CS)**
Erfinder: **Prikryl, Ivan, MUDr**
**Sypka 15**
**Brno(CS)**

(74) Vertreter: **Patentanwälte Beetz sen. - Beetz**
**jun. Timpe - Siegfried - Schmitt-Fumian**
**Steinsdorfstrasse 10**
**D-8000 München 22(DE)**

(54) **Hämostatisches Material und seine Herstellung.**

(57) Die Erfindung betrifft ein hämostatisches Material auf der Basis faserförmiger Teilchen eines Hämostatikums sowie Verfahren zu seiner Herstellung.

In dem hämostatischen Material liegt das Hämostatikum in einer mit einem Adhäsionsmittel räumlich vernetzten Struktur vor. Bevorzugte Hämostatika sind Carboxycellulose, mikrokristallines Collagen, Alginsäure sowie deren Alkali-und Calciumsalze; bevorzugte Adhäsionsmittel sind Methylcellulose, Hydroxyethylcellulose, Methylhydroxyethylcellulose und Methylhydroxypropylcellulose, wobei vorzugsweise 1 bis 10 Teilchen des Adhäsionsmittels auf 1 Teilchen des hämostatischen Mittels entfallen, was einem Gehalt von mindestens 1 Masse-% Adhäsionsmittel entspricht.

Das erfindungsgemäße hämostatische Material ergibt eine gesteigerte Geschwindigkeit bei der Blutstillung von Kapillarblutungen, wobei die mechanischen Eigenschaften zugleich einen Kompressionseffekt in blutenden Wunden verursachen und damit die Blutstillung selbst noch unterstützen.

Die hämostatischen Materialien sind biologisch resorbierbar und entsprechen vom pharmakologischen Gesichtspunkt den an lokalwirkende Hämostatika zu stellenden Ansprüchen und sind auch hinsichtlich der Herstellung der verschiedenen Anwendungsformen, insbesondere für die Stomatologie und die Otorhinolaryngologie, vorteilhaft.

## Hämostatisches Material und seine Herstellung

Die Erfindung betrifft ein hämostatisches Material auf der Basis faserförmiger Teilchen mindestens einer im Kontakt mit Blut hämostatisch wirkenden Substanz, wie insbesondere Carboxycellulose und/oder deren Alkali-und/oder Calciumsalze, mikrokristallines Collagen und/oder Alginsäure und/oder deren Alkali-und/oder Calciumsalze als Hämostatika, sowie Verfahren zur Herstellung des hämostatischen Materials.

In der medizinischen Praxis kommen venöse Kapillarblutungen aus den verschiedensten Ursachen relativ häufig vor. Zur Sicherstellung eines günstigen Heilungsverlaufs ist in diesen Fällen vor allem eine Blutstillung erforderlich. Dabei ist es neben der gesamten Therapie zumeist nötig, direkt in die blutende Wunde einzugreifen. Ein für diese Zwecke geeignetes Lokalhämostatikum sollte eine hohe hämostatische Wirksamkeit sowie ausgeprägtes Sorptionsvermögen aufweisen, wobei gleichzeitig die Forderung nach einer gewissen mechanischen Festigkeit und Kohäsion erfüllt sein sollte, um eine Kompression des blutenden Feldes zu ermöglichen. Darüber hinaus wird in vielen Fällen auch eine Formbarkeit des Hämostatikums unter der Einwirkung von mäßigem Druck gefordert. Das hämostatische Material soll ferner in verschiedenen Anwendungsformen, wie etwa in Form von Folien, Platten, Verbänden, profilierten Formkörpern und ggfs. dreidimensionalen Gebilden, für eine zweckmäßige Applikation verfügbar sein.

Im Hinblick auf die Anwendungsbedingungen muß das hämostatische Material ferner hygienisch einwandfrei, ohne Irritationswirkung und steril sein und ggfs. auch vom Gewebe des Organismus resorbierbar sein. Darüber hinaus können auch antimikrobielle, bakterizide bzw. bakteriostatische wie auch lokalanästhetische Eigenschaften gefordert sein.

Hämostase im üblichen Sinne ist das Ergebnis einer Kette enzymatischer Reaktionen, welche die Koagulation des Fibrinogens im Blutplasma verursachen. Die Polymerisation des Fibrinogens zu Fibrin und die nachfolgende Vernetzung der faserigen Fibrinstruktur hat eine Erhöhung der mechanischen Festigkeit und der Elastizität des resultierenden Thrombus zur Folge. Das Prinzip der Wirkungsweise von Lokalhämostatika ist bisher nur in groben Zügen bekannt. Im allgemeinen wird angenommen, daß beispielsweise Carboxycellulose, die ein Lokalhämostatikum darstellt, im wesentlichen nicht aktiv in das Koagulationssystem eintritt. Durch Laboruntersuchungen wurde festgestellt, daß Carboxycellulose das Adhäsionsvermögen von Erythrocyten im venösen Nativblut erhöht, deren Aggregation beschleunigt und ihre Bindung an die faserförmigen Teilchen der Carboxycellulose verursacht, so daß sich schließlich ein kombinierter, die Stellung der Kapillarblutung bewirkender Thrombus bildet (vgl. T. T. Daurova et al., Klinická chirurgie 1981, Nr. 1, S. 5 bis 7).

Unter diesem Gesichtspunkt kann die hämostatische Wirksamkeit von Carboxycellulose bei Patienten mit Blutkoagulationsstörungen erklärt werden. Die von Zellgewebe resorbierbaren Lokalhämostatika sind ferner für klinische Zwecke besonders vorteilhaft, da sie im Organismus in situ belassen werden können.

Zu den lokalwirkenden Hämostatika gehören bestimmte hydrophile Naturpolymere (Polynosics) und ihre Derivate. Durch selektive Oxidation der primären alkoholischen Gruppen etwa von Baumwolle oder Regeneratcellulose wird eine lokalhämostatisch wirksame Carboxycellulose erhalten, die genau genommen ein Copolymerisat von Anhydroglycose und Anhydroglucuronsäure darstellt, die zumeist 12 bis 24 % Carboxylgruppen enthält, die teilweise oder ganz in kationischer Salzform vorliegen können. Derartige Carboxycellulosen sind durch Zellgewebe resorbierbar und können in Form von Geweben oder gewirkten bzw. gestrickten Materialien eingesetzt werden.

Von den Celluloseethern weisen Carboxymethylcellulose und Carboxypropylcellulose lokalhämostatische Wirksamkeit auf. Bei Kontakt dieser Substanzen mit venösem Blut treten Quellung und Übergang in einen gelartigen Zustand auf. Die Alkalisalze von Carboxymethylcellulosen und Carboxypropylcellulosen sind bereits vollständig löslich und besitzen keine hämostatische Wirksamkeit mehr.

Resorbierbare, von Collagen abgeleitete Lokalhämostatika sind ebenfalls von großer Bedeutung. Die durch Lyophilisierung entsprechender Hydrogele erhältlichen Hämostatika besitzen eine - schaumartige Struktur. Das mikrofibrilläre Collagen wird dabei durch Einwirkung verdünnter Säuren auf Collagen und mechanische Zerkleinerung zu faserigen Teilchen gewonnen. Eine Erhöhung der hämostatischen Wirksamkeit kann durch Veresterung und Vernetzung durch Zusatz von Dialdehyden erzielt werden.

Zu den Proteinen gehört auch der lokalhämostatisch wirksame Fibrinschaum, der durch Fraktionierung von Humanplasma erhalten und gewöhnlich zusammen mit Thrombin angewandt wird.

Die indirekte lokalhämostatische Wirkung von alpha-Cyanoacrylaten tritt aufgrund ihrer Polymerisation bei Kontakt mit Zellgeweben ein. Der daraufhin entstehende Belag an der Wunde verursacht eine mechanische Blutstillung.

Als Lokalhämostatika wurden ferner auch Salze von Polyacrylsäuren empfohlen, beispielsweise die Calcium-und Eisen(III)-Salze, die in Form einer Beschichtung auf Verbandmaterialien angewandt wurden.

Eine besondere Gruppe bilden modifizierte Arten von Lokalhämostatika. Gemäß den US-PSen 2 914 444 und 3 122 479 können zusammengesetzte hämostatische, von freier Carboxymethylcellulose, Carboxypropylcellulose und Carboxycellulose ableitbare Materialien hergestellt werden. Als Weichmacher werden dabei Glycerin, Sorbit und Polyethylenglycole angegeben. Entsprechende Applikationsformen sind Flachmaterialien, leichte Schichtmaterialien, Fasern, Pulver, Stäbchen, Gele und Pasten.

Ein weiteres Beispiel für modifizierte Lokalhämostatika sind die Formen von Carboxycellulosen, die durch Mischen mit einer wäßrigen Lösung eines Bindemittels, wie beispielsweise des Natriumsalzes von Carboxymethylcellulose, erhältlich sind. Nach dem Trocknen entsprechender Gemische können flächige oder dreidimensionale Formkörper gewonnen werden. Nach Zugabe eines größeren Anteils eines polaren Weichmachers, beispielsweise Glycerin, werden plastische Materialien mit hämostatischen Eigenschaften erhalten, die sich z. B. als Knochenbindemittel eignen.

Die oben erläuterten Arten der verschiedenen bekannten Lokalhämostatika weisen neben den günstigen Applikationseigenschaften auch einige Nachteile auf. Die pulverförmigen Lokalhämostatika, die Fasergebilde sowie die lokalhämostatischen Gewebematerialien schließen die Möglichkeit einer Kompression einer blutenden Wunde wegen des fehlenden mechanischen Zusammenhalts und entsprechender Festigkeit aus. Die Blutdurchlässigkeit dieser bekannten Lokalhämostatika erlaubt ferner keine Ausnutzung als Diffusionsblutungssperre. Die aus einem leichten Schaum bestehenden Arten aus Collagen hergestellter Lokalhämostatika verlieren ferner unmittelbar nach dem Kontakt mit Nativblut ihre Festigkeit und ihren Zusammenhalt.

Der Erfindung liegt entsprechend die Aufgabe zugrunde, unter Vermeidung der Nachteile der bisher bekannten Hämostatika ein hämostatisches Material und seine Herstellung anzugeben, mit dem eine Kapillarblutstillung, insbesondere nach Verletzungen und chirurgischen Eingriffen, möglich ist, wobei die Struktur und die Eigenschaften des hämostatischen Materials eine erhöhte lokalhämostatische Wirksamkeit, ein hohes Sorptionsvermögen und gleichzeitig die erforderliche Undurchlässigkeit für Blut ergeben sollen, wobei ferner die mechanische Festigkeit und Steifigkeit eine Kompression blutender Oberflächen ermöglichen sollen.

Die Aufgabe wird gemäß den Ansprüchen 1, 31 bzw. 52 gelöst; vorteilhafte Ausführungsformen sind Gegenstand der Unteransprüche.

Erfindungsgemäß ist vorteilhaft, daß die hämostatischen Materialien eine vernetzte Raumstruktur aufweisen, die eine leichte Herstellung verschiedenster Formkörper je nach Zweck und Anwendungsfall ermöglicht.

Das erfindungsgemäße hämostatische Material beruht auf der Basis faserförmiger Teilchen mindestens einer im Kontakt mit Blut hämostatisch wirkenden Substanz als Hämostatikum und ist dadurch gekennzeichnet, daß die faserförmigen Teilchen des Hämostatikums mit Teilchen eines Adhäsionsmittels zu einer räumlich vernetzten Struktur physikalisch bzw. physikochemisch gebunden sind.

Geeignete Hämostatika sind insbesondere Carboxycellulose und/oder deren Alkali-bzw. Calciumsalze, kristallines Collagen sowie Alginsäure und/oder deren Alkali-bzw. Calciumsalze.

Geeignete Adhäsionsmittel sind insbesondere Methylcellulose, Hydroxyethylcellulose, Hydroxypropylcellulose, Methylhydroxyethylcellulose und Methylhydroxypropylcellulose.

Günstigerweise wird das Adhäsionsmittels so eingesetzt, daß 1 bis 10 Teilchen des Adhäsionsmittels auf ein Teilchen des Hämostatikums entfallen.

Durch Kombination verschiedener Lokalhämostatika mit unterschiedlichen Eigenschaften läßt sich für manche Anwendungsfälle eine noch bessere Wirksamkeit erzielen. Dies gilt insbesondere für das Problem einer Beschleunigung der Aktivierung der Thrombusbildung und der daraufhin erfolgenden Stillung der Kapillarblutung, der Erhöhung der Blutsorptionsgeschwindigkeit, der Erzielung ausreichender mechanischer Festigkeit sowie der erforderlichen Undurchlässigkeit des Hämostatikums. Die Erfindungskonzeption umfaßt entsprechend auch hämostatische Materialien, die mehrere verschiedene Arten faserförmiger Teilchen von Lokalhämostatika enthalten, die durch die Teilchen des Adhäsionsmittels gebunden sind.

Für hämostatische Formkörper, insbesondere stomatologische Zäpfchen, ist es erforderlich, neben einer schnellen Aktivierung der Thrombusbildung auch eine mechanische Festigkeit sowie Undurchlässigkeit für das aus dem Alveolus nach einer Zahnextraktion entweichende Blut sicherzustellen. Diese sehr unterschiedlichen Erfordernisse werden z. B. durch Kombination von faserförmigen Teilchen des Calciumsalzes von Carboxycellulose mit faserförmigen Teilchen von Alginaten und/oder Collagen erfüllt, da hierdurch das hämostatische Material nach der Sättigung mit Blut die gewünschte Undurchlässigkeit erhält.

Das erfindungsgemäße hämostatische Material besitzt eine physikalisch vernetzte Raumstruktur, bei der die faserförmigen Teilchen des Hämostatikums mit Hilfe der Teilchen des Adhäsionsmittels punktuell aneinander gebunden sind. Diese Struktur entsteht, wenn im statistischen Mittel auf ein Teilchen des faserförmigen Hämostatikums 1 bis 10 Teilchen des Adhäsionsmittels entfallen. Eine ideale Raumnetzstruktur würde sich streng genommen unter diesen Bedingungen nur dann bilden, wenn sämtliche Teilchen des fa serförmigen Hämostatikums gleiche Länge aufweisen. In der Praxis besitzen jedoch die faserförmigen Teilchen des Hämostatikums in der Regel unterschiedliche Länge, was durch eine entsprechende Verteilungskurve zum Ausdruck gebracht werden kann. In diesen Fällen ist es notwendig, die Anzahl der Wechselwirkungsstellen und damit auch das oben genannte Zahlenverhältnis zu Gunsten der Teilchen des Adhäsionsmittels zu ändern. Bei einer breiten Verteilungskurve der Größe der faserförmigen Teilchen sind bei der Ausbildung der vernetzten Raumstruktur Teilchen mit größerer Länge bevorzugt. Kleine faserförmige Teilchen werden jedoch dabei im räumlichen Netzwerk eingeschlossen und fixiert.

Durch statistische Betrachtungen und experimentelle Untersuchungen wurden die Bedingungen ermittelt, unter denen es zur Bildung einer zusammenhängenden, mechanisch bzw. physikalisch vernetzten Struktur des hämostatischen Materials kommt. Sie entsteht dann, wenn auf ein Teilchen des faserförmigen Hämostatikums mindestens ein Teilchen des Adhäsionsmittels entfällt. Dabei kann der Massenanteil der Teilchen des Adhäsionsmittels, der dem genannten kritischen Verhältnis entspricht, durch Verminderung ihrer Größe bis auf 1 Masse-% herabgesetzt werden. Durch Erhöhung des Mengenanteils des Adhäsionsmittels steigt die mechanische Festigkeit und gleichzeitig auch die Steifigkeit der vernetzten Raumstruktur des hämostatischen Materials an.

Die biologischen Eigenschaften des erfindungsgemäßen hämostatischen Materials und insbesondere die hämostatische Wirksamkeit bei der lokalen Anwendung sowie die Absorbierbarkeit bzw. Resorbierbarkeit wie auch vom Standpunkt der Anwendung bedeutsame physikalische bzw. mechanische Eigenschaften, wie etwa die Dichte, die mechanische Festigkeit, die Biegsamkeit, die Komprimierbarkeit und Formbarkeit, sowie ferner die Hydrophilie, die Geschwindigkeit der Sorption polarer Flüssigkeiten und die Sorptionskapazität können je nach Anwendungszweck und vorgesehenem Anwendungsort im Organismus innerhalb breiter Bereiche modifiziert und eingestellt werden. Struktur und Eigenschaften des erfindungsgemäßen Hämostatikums hängen dabei vor allem von der zweckmäßigen Wahl und den relativen Mengenverhältnissen der Teilchen des faserförmigen Hämostatikums und des Adhäsionsmittels innerhalb des breiten, oben angegebenen Bereichs sowie von der jeweiligen Teilchengröße ab. Auf dieser Basis ist es möglich, die Eigenschaften des Hämostatikums auch durch Modifizieren einiger Verfahrensschritte bei der Herstellung verschiedener Applikationsformen zu ändern und einzustellen.

Die faserförmigen Teilchen des Hämostatikums beruhen auf der Basis oxidierter Cellulose bzw. Carboxycellulose und sind durch Zellgewebe absorbierbar und resorbierbar. Die Carboxylgruppen, die vorzugsweise in einer Konzentration von 8 bis 24 % vorliegen, können teilweise oder vollständig in Form von Salzen mit Kationen vorliegen.

Die faserförmigen Teilchen des Hämostatikums können ferner auf der Basis eines aus tierischen Produkten auf chemischem Wege gewonnenen mikrokristallinen Collagens beruhen oder aus Alginsäure hergestellt werden, die ggfs. teilweise oder ganz zum entsprechenden Alkali-oder Calciumsalz neutralisiert wird.

Die erfindungsgemäß zur Bindung der Teilchen des Hämostatikums in einer räumlich vernetzten Struktur herangezogenen Adhäsionsmittel sind Materialien auf der Basis von Methylcellulose, Hydroxyethylcellulose, Hydroxypropylcellulose, Methylhydroxyethylcellulose oder Methylhydroxy propylcellulose, deren mittlerer Substitutionsgrad 0,5 bis 1,5 beträgt, wobei die Materialien eine Reinheit von mindestens 99,5 % besitzen. Diese Substanzen sind ebenfalls durch Zellgewebe absorbierbar und resorbierbar.

In die vernetzte Raumstruktur mit den faserförmigen Teilchen können ferner in Blut rasch quellende und hohes Sorptionsvermögen aufweisende Teilchen verschiedener Art eingebracht werden, die in der vernetzten Raumstruktur adhäsiv fixiert sind.

Derartige schnell quellende Teilchen mit hohem Sorptionsvermögen, insbesondere für Blut, werden z. B. insbesondere aus Carboxymethylstärke und/oder Hydroxyethylstärke hergestellt. Der mittlere Substitutionsgrad dieser Materialien beträgt günstigerweise 0,5 bis 1,5. Diese Teilchen sind ebenfalls vom Zellgewebe des Organismus absorbierbar und resorbierbar.

Die faserförmigen Teilchen des Hämostatikums, wie beispielsweise Carboxycellulose, können mit bis zu 50 % ihrer Masse mit einem polaren Weichmacher, wie insbesondere Polyethylenglycol mit einer mittleren Molekülmasse von 150 bis 600, oder Glycerin imprägniert werden, wodurch die Biegefähigkeit und Formbarkeit erhöht wird. Der Zusatz einer übermäßigen Menge an Weichmacher übt jedoch eine ungünstige Wirkung auf die Beschleunigung der Hämostase aus und vermindert ferner die Sorptionskapazität des hämostatischen Materials. Im Hinblick darauf ist eine optimale Dosierung des Weichmachers je nach den für den betreffenden Anwendungsfall geforderten mechanischen Eigenschaften des Produkts von Bedeutung.

Das erfindungsgemäße Verfahren zur Herstellung des Hämostatikums beruht auf dem gleichmäßigen Dispergieren oder Einmischen des Adhäsionsmittels in das Hämostatikum, wobei es günstig ist, das Hämostatikum vor dem Einbringen des Adhäsionsmittels mit dem polaren Weichmacher zu imprägnieren oder in ihm zu dispergieren.

Dem Hämostatikum kann zur Erleichterung der Herstellung ein hygienisch bzw. pharmakologisch geeigneter Emulgator, beispielsweise destilliertes Palmitinsäure-und/oder Stearinsäuremonoglycerid oder ethoxyliertes Sorbitoleat, in einer Menge von bis zu 5 Masse-%, bezogen auf die Masse des Hämostatikums, zugegeben werden.

Das erfindungsgemäße homostatische Material mit räumlich vernetzter Struktur kann ferner auch als Träger für pharmazeutische Wirkstoffe dienen, die durch Adhäsion, Chemisorption oder chemische Bindung an reaktive funktionelle Gruppen des Hämostatikums und/oder des Adhäsionsmittels und/oder andere in der Matrix des hämostatischen Materials enthaltene Additive gebunden sind. Beispiele für geeignete pharmazeutische Wirkstoffe sind Lokalanästhetika, wie Cinchocain, Benzocain, Mesocain und Lidocain. Die pharmazeutischen Wirkstoffe können in der räumlich vernetzten Matrix des hämostatischen Materials in einer Menge von bis zu 1 Masse-% vorliegen und sind vorzugsweise in der Matrix des hämostatischen Materials verteilt.

Das erfindungsgemäße homostatische Material kann ferner auch Zusätze mit antimikrobieller bzw. bakterizider und/oder bakteriostatischer Wirkung enthalten, z. B. Methylparaben oder Alkalisalze der Sorbinsäure, die in einer Menge von bis zu 5 Masse-%, bezogen auf das Hämostatikum vorliegen können. Geeignete bakterizide Wirkstoffe sind solche vom kationoiden Typ, wie Cetyltrimethylammoniumbromid und Carbethoxypentadecyltrimethylammoniumbromid, die an freien Carboxylgruppen der als Hämostatikum vorliegenden Carboxycellulose chemisch gebunden werden können. Als Bakteriostatika eignen sich ferner Sorbinsäure und deren Alkalisalze.

Aus dem erfindungsgemäßen hämostatischen Material mit räumlich vernetzter Struktur können die verschiedensten Formkörper hergestellt werden, deren geometrische Form an den jeweiligen Anwendungszweck angepaßt ist, beispielsweise Folien, Platten und Verbände bzw. Flachmaterialien, die zur Stillung von Kapillarblutungen an der Körperoberfläche oder in einzelnen inneren Organen bestimmt sind, ferner spezielle Formkörper wie Nasen-und Zahnzäpfchen.

Flache Formkörper werden günstigerweise ein- oder beiderseitig mit einem Dessin versehen. Das zur Blutstillung in Körperhöhlen bestimmte hämostatische Material kann ferner je nach dem betreffenden Anwendungsfall zu Profilen verschiedenen Querschnitts verarbeitet werden.

Das erfindungsgemäße hämostatische Material kann ferner auch Bestandteil eines aus mehreren Schichten bestehenden und zum Aufbringen auf blutende Wunden bestimmten flachen Verbandmaterials oder Mehrkomponenten-Tampons zur Hämostase in Körperhöhlen sein.

Die Erfindungskonzeption beruht auf dem Ergebnis von Forschungsarbeiten, die sich mit den Problemen der Thrombogenese in venösem Nativblut und seiner Veränderungen unter dem Einfluß hydrophiler natürlicher und synthetischer Polymerisate beschäftigten. Durch Thromboelastographie wurde nachgewiesen, daß einige modifizierte natürliche Polymere aus der Gruppe der Celluloseether die Thrombusbildung in venösem Nativblut in beträchtlichem Ausmaß aktivieren und somit die Stillung von Kapillarblutungen beschleunigen. Die Aufklärung des zugrundeliegenden Mechanismus dieser Kontaktwirkung ist Gegenstand weiterer Forschung und derzeit noch nicht abschließend geklärt. Ausgewählte Typen von Celluloseethern weisen gleichzeitig sehr gute Adhäsionseigenschaften gegenüber den faserförmigen Teilchen der Carboxycellulose, des Collagens oder Alginaten auf, so daß sie zur Erzielung einer räumlich vernetzten Struktur des hämostatischen Materials eingesetzt werden können. Durch Laboruntersuchungen und klinische Tests wurde nachgewiesen, daß beispielsweise eine geeignet gewährte Strukturkombination faserförmiger Teilchen von Carboxycellulose mit ausgewählten Celluloseethern zu einer Erhöhung der hämostatischen Wirksamkeit führt.

Neben der raschen Stillung von Kapillarblutungen ist bei dem erfindungsgemäßen hämostatischen Material auch das Sorptionsvermögen für Blut von Bedeutung, und zwar sowohl vom Standpunkt der Sorptionsgeschwindigkeit als auch dem der Sorptionskapazität. Diese Eigenschaft des hämostatischen Materials hängt mit der Kinetik der Quellung der entsprechenden Polymerisate in wäßrigen Lösungen zusammen. Eine hohe Quellungsgeschwindigkeit und gleichzeitig eine hohe Sorptionskapazität gegenüber Blut weisen beispielsweise Carboxymethylstärke, Hydroxyethylstärke und vernetzte Carboxymethylcellulose auf. Durch Verwendung dieser Komponenten im erfindungsgemäßen homostatischen Material auf der Basis faserförmiger Teilchen von Carboxycellulose ist eine ausgeprägte Verbesserung des Sorptionsvermögens erzielbar.

Der Einfluß des zugesetzten Sorptionsmittels auf die Ge schwindigkeit der Thrombusbildung in venösem Nativblut wurde durch thromboelastographische Messungen untersucht. Dabei wurde festgestellt, daß die genannten Komponenten per se zwar keine besondere hämostatische Wirksamkeit zeigen, jedoch in Kombination mit den faserförmigen Teilchen von Carboxycellulose, mikrofibrillärem Collagen oder Alginaten und Teilchen von Methylcellulose oder Methylhydroxyethylcellulose als Adhäsionsmittel die hämostatische Gesamtwirkung auch nicht verringern.

Sämtliche oben erwähnten notwendigen und fakultativen Bestandteile des erfindungsgemäßen hämostatischen Materials sind aus hygienischer Sicht einwandfrei und durch Zellgewebe absorbierbar und resorbierbar.

Das erfindungsgemäße, hämostatische Material kann in einer gasdichten Verpackung mit ionisierender Strahlung bei einer Energiedosis von 25 kGy (25 kJ/kg) sterilisiert werden. Durch die Strahlungssterilisation verändern sich die ausgenutzten charakteristischen Eigenschaften des hämostatischen Materials nicht, insbesondere die hämostatische Wirksamkeit, die Geschwindigkeit der Sorption von Blut und die Sorptionskapazität.

Das erfindungsgemäße hämostatische Material ist sowohl aufgrund seiner einfachen Herstellbarkeit als auch der günstigen Applikationseigenschaften mit einer Reihe großer Vorteile verbunden, wie aus der obigen Erläuterung hervorgeht. So können insbesondere venöse Kapillarblutungen mit dem hämostatischen Material außerordentlich rasch gestillt werden, wobei zugleich eine hohe Sorptionskapazität für Blut vorliegt.

Die in der Struktur des erfindungsgemäßen hämostatischen Materials vorliegenden Poren - schließen sich nacheinander aufgrund der Sorption von Blut und der Quellung der Teilchen des hydrophilen Sorptionsmittels, so daß das hämostatische Material dann als Diffusionssperre für die weitere Blutung zu dienen beginnt. Durch die ausreichende mechanische Festigkeit der angequollenen Struktur des räumlich vernetzten erfindungsgemäßen hämostatischen Materials wird ferner eine Kompressionswirkung in blutenden Wunden ermöglicht, wodurch die Blutstillung ebenfalls unterstützt wird.

Das erfindungsgemäße hämostatische Material und seine Herstellung werden im folgenden anhand von Ausführungsbeispielen näher erläutert.

Beispiel 1

Aus faserförmigen Teilchen von Carboxycellulose mit 14 % Carboxylgruppen in Form des entsprechenden Calciumsalzes wurde ein hämostatisches Flachmaterial mit räumlich vernetzter Struktur hergestellt. Die Teilchengrößenverteilung der faserförmigen Teilchen des Hämostatikums lag im Bereich von 30 bis 500 μm, wobei die faserförmigen Teilchen statistisch im Raum orientiert und zum Teil in der Ebene des Flachmaterials ausgerichtet waren. Der Mengenanteil des Hämostatikums betrug dabei 75 Masse-%.

Die faserförmigen Teilchen des Calciumsalzes der Carboxycellulose wurden mit einem polaren Weichmacher, wie Polyethylenglycol mit einer mittleren Molekülmasse von 300 imprägniert, dessen Mengenanteil 10 Masse-%, bezogen auf das hämostatische Material, betrug. In der Struktur wurden dann im Mittel 50 μm große Teilchen von Methylhydroxyethylcellulose als Adhäsionsmittel gleichmäßig disper giert, die in einer relativen Menge von 15 Masse-% vorlagen und die faserförmigen Teilchen des Hämostatikums zu einer vernetzten Raumstruktur verbanden. Hierdurch erhielt das hämostatische Flachmaterial die erforderliche mechanische Festigkeit unter Beibehaltung der notwendigen Biegsamkeit. Das Zahlenverhältnis der Teilchen des Hämostatikums zu den Teilchen des Adhäsionsmittels betrug etwa 1:1,5.

Beispiel 2

Aus faserförmigen Teilchen des Calciumsalzes von Carboxycellulose mit einem Carboxylgruppengehalt von 14 % wurde ein hämostatisches Flachmaterial hergestellt, wobei die Teilchenlängenverteilung im Bereich von 30 bis 500 μm lag. Die faserförmigen Teilchen des Hämostatikums lagen in einer Menge von 66 Masse-%, bezogen auf die Gesamtmasse des Produkts, vor. Sie wurden mit einem polaren Weichmacher (Polyethylenglycol mit einer mittleren Molekülmasse von 300, Mengenanteil 10 Masse-%) weichgemacht. Die Fasern des Hämostatikums waren teilweise in mit der Oberfläche des Flachmaterials parallelen Ebenen orientiert.

In der Struktur des Hämostatikums wurden im Mittel 50 μm große Teilchen von Methylhydroxyethylcellulose in einer Menge von 12 Masse-% gleichmäßig dispergiert, wobei das Zahlenverhältnis der Teilchen des Hämostatikums zu den Teilchen des Adhäsionsmittels etwa 1:1,5 betrug. Durch die Teilchen des Adhäsionsmittels wurden die faserförmigen Teilchen des Hämostatikums miteinander zu einer räumlich vernetzten Struktur verbunden. Auf dieselbe Weise wie das Adhäsionsmittel wurden hydrophile Teilchen von Carboxymethylstärke in einer Menge von 12 Masse-% in die Struktur des hämostatischen Materials eingebracht, die stark quellen und im Kontakt mit dem hämostatischen Material eine rasche Sorption von Blut ergeben.

Das erhaltene modifizierte hämostatische Flachmaterial wies eine sehr hohe Blutstillungswirksamkeit sowie erhöhtes Sorptionsvermögen auf, wobei gleichzeitig die geforderte mechanische Festigkeit vorlag.

### Beispiel 3

Aus faserförmigen Teilchen von Carboxycellulose mit 18 % freien Carboxylgruppen - (Teilchengröße 200 bis 600 $\mu$m; Massenanteil 79,5 Masse-%) wurde ein hämostatisches Flachmaterial mit räumlich vernetzter Struktur hergestellt. Die Teilchen des Hämostatikums wurden mit einem Polyethylenglycol mit einer mittleren Molekülmasse von 300 weichgemacht, das in einer Menge von 11,3 Masse-% eingesetzt wurde. Danach wurden Teilchen von Hydroxyethylcellulose mit einer mittleren Teilchengröße von 40 $\mu$m in einer Menge von 6,6 Masse-% gleichmäßig im hämostatischen Flachmaterial dispergiert, wobei das Zahlenverhältnis der Teilchen des Hämostatikums zu den Teilchen des Adhäsionsmittels etwa 1:2 betrug.

Aufgrund der Teilchen des Adhäsionsmittels wurden die faserförmigen Teilchen des Hämostatikums zu einer räumlich vernetzten Struktur verbunden. Die Gleichmäßigkeit der Dispersion der Teilchen des Adhäsionsmittels wurde durch Verwendung eines hygienisch geeigneten Emulgators (Sorbitoleat) gefördert, der in einer Menge von 2,6 Masse-% eingesetzt wurde.

### Beispiel 4

Aus faserförmigen Teilchen von Carboxycellulose mit 18 % freien Carboxylgruppen (Teilchengröße 50 bis 500 $\mu$m; Menge 65 Masse-%) wurde ein hämostatisches Flachmaterial hergestellt. Zur Verbesserung der Biegsamkeit wurden die Teilchen des Hämostatikums mit 15 Masse-% eines Polyethylenglycols mit einer mittleren Molekülmasse von 300 weichgemacht und dann mit Teilchen von Hydroxyethylcellulose (mittlere Teilchengröße 50 $\mu$m; Mengenanteil 15 Masse-%) als Adhäsionsmittel miteinander zu einer räumlich vernetzten Struktur verbunden, wobei das Zahlenverhältnis der Teilchen des Hämostatikums zu den Teilchen des Adhäsionsmittels etwa 1:1,5 betrug. Die Dispersion der beiden Komponenten wurde durch Zusatz eines hygienisch geeigneten Emulgators (destilliertes Palmitinsäure-und Stearinsäuremonoglycerid) gefördert, der in einer Menge von 5 Masse-% eingesetzt wurde.

### Beispiel 5

Aus faserförmigen Teilchen von Carboxymethylcellulose mit 16 % freien Carboxylgruppen wurde ein hämostatisches Flachmaterial hergestellt, wobei etwa 50 % der Carboxylgruppen als solche vorlagen, und 50 % durch partielle Neutralisation zum entsprechenden Natriumsalz umgewandelt waren. Zur Erhöhung der Biegsamkeit wurden die Teilchen des eingesetzten Hämostati kums, deren Teilchengröße im Bereich von 50 bis 500 $\mu$m lag, durch Sorption von Glycerin als hygienisch geeignetem polaren Weichmacher modifiziert, der in einer Menge von 19,4 Masse-% eingesetzt wurde. Durch Zusatz von Teilchen von Hydroxypropylcellulose mit einer mittleren Teilchengröße von 60 $\mu$m in einer Menge von 17,5 Masse-% wurde das Hämostatikum zu einer vernetzten Raumstruktur gebunden, wobei das Zahlenverhältnis der Teilchen des Hämostatikums zu den Teilchen des Adhäsionsmittels etwa 1:2,5 betrug. Die Dispergierung der beiden Komponenten wurde durch Zusatz von Palmitinsäure-und Stearinsäuremonoglycerid als Emulgator in einer Menge von 4,8 Masse-% gefördert.

### Beispiel 6

Aus nichtorientierten faserförmigen Teilchen des Calciumsalzes von Carboxycellulose mit 14 % freien Carboxylgruppen, das in einer Menge von 75 Masse-% eingesetzt wurde, wurden hämostatische Formkörper in Form von zur Blutstillung nach Zahnextraktion bestimmten Zäpfchen hergestellt. Diese Teilchen wurden mit einem Polyethylenglycol mit einer mittleren Molekülmasse von 300 als polarem Weichmacher weichgemacht, der in einem Mengenanteil von 17 Masse-% eingesetzt wurde. Durch Zusatz von Teilchen von Methylhydroxyethylcellulose einer mittleren Teilchengröße von etwa 50 $\mu$m (Reinheit 99,5 %), die in einer Menge von 7,5 Masse-% eingesetzt wurden, wurde eine entsprechende räumlich vernetzte Struktur aufgebaut. Das Zahlenverhältnis der Teilchen des Hämostatikums zu den Teilchen des Adhäsionsmittels betrug etwa 1:2.

Auf die Oberfläche der faserförmigen Teilchen des Hämostatikums wurde ein Lokalanästhetikum (Mesocain) in einer Menge von 0,5 Masse-% aufgebracht. Die aus dem so erhaltenen räumlich vernetzten hämostatischen Material hergestellten hämostatischen Zäpfchen für stomatologische Zwecke zeichneten sich durch rasche Blutstillung im Zahnalveolus aus und waren aufgrund der geeigneten mechanischen Festigkeit komprimierbar.

Beispiel 7

Aus nichtorientierten faserförmigen Teilchen des Calciumsalzes von Carboxycellulose mit 14 % freien Carboxylgruppen, die eine Teilchengröße im Bereich von 100 bis 500 μm besaßen und in einer Menge von 70 Masse-% eingesetzt wurden, wurden hämostatische, zur Blutstillung nach Zahnextraktion bestimmte Zäpfchen mit erhöhtem Sorptionsvermögen hergestellt. Durch Imprägnierung des Hämostatikums mit einem Polyethylenglycol mit einer mittleren Molekülmasse von 300 als polarem Weichmacher, der in einer Menge von 14 Masse-% eingesetzt wurde, wurden die Teilchen des Hämostatikums zunächst weichgemacht, worauf Teilchen von Methylhydroxyethylcellulose einer mittleren Teilchengröße von etwa 50 μm in einer Menge von 7,5 Masse-% als Adhäsionsmittel eingebracht und mit den Teilchen des Hämostatikums homogenisiert wurden, wobei das Zahlenverhältnis der Teilchen des Hämostatikums zu den Teilchen des Adhäsionsmittels etwa 1:1,8 betrug. Ebenso wie die Teilchen des Adhäsionsmittels wurden ferner in der Zäpfchenmasse Teilchen von hydrophiler, in Blut quellender Carboxymethylstärke mit einer Teilchengröße im Bereich von 50 bis 100 μm in einer Menge von 8 Masse-% gleichmäßig dispergiert.

Auf den faserförmigen Teilchen des Calciumsalzes der Carboxycellulose wurde ein Lokalanästhetikum (Lidocain) in einer Menge von 0,5 Masse-% aufgebracht. Die so erhaltenen modifizierten hämostatischen Zäpfchen zeichneten sich neben rascher Blutungsstillung und Komprimierbarkeit unter Druckeinwirkung vor allem auch durch erhöhte Sorptionsgeschwindigkeit gegenüber Blut und einen erhöhten Sorptionsgrad aus.

Beispiel 8

Aus faserförmigen Teilchen von Carboxycellulose mit 18 % freien Carboxylgruppen und einer mittleren Teilchengröße (Länge) von 300 μm, die in einer Menge von 72 Masse-% eingesetzt wurden, wurden hämostatische Zäpfchen zur Stillung von Postextraktionsblutungen in der Stomatologie hergestellt. Durch Sorption eines hydrophilen, hygienisch geeigneten polaren Weichmachers - (Glycerin), der in einer Menge von 8 Masse-% eingesetzt wurde, wurden die Teilchen der Carboxycellulose weichgemacht. Durch Zusatz von Teilchen von Hydroxyethylcellulose einer mittleren Teilchengröße von 65 μm, die in einer Menge von 11,8 Masse-% eingesetzt wurden, wurde eine entsprechende räumlich vernetzte Struktur aufgebaut. Das Zahlenverhältnis der Teilchen des Hämostatikums zu den Teilchen des Adhäsionsmittels betrug etwa 1:1,5. Zur Steigerung des Sorptionsvermögens wurden ferner 8 Masse-% Hydroxyethylstärke und als Bakterizid 0,2 Masse-% Cetyltrimethylammoniumbromid zugegeben.

Beispiel 9

Aus faserförmigen Teilchen auf der Basis von Alginsäure, die zu 80 % in das Calciumsalz umgewandelt war und eine mittlere Teilchengröße - (Länge) von 800 μm aufwies und in einer Menge von 82 Masse-% eingesetzt wurde, wurden hämostatische Zäpfchen für stomatologische Applikationszwecke hergestellt. Das Hämostatikum wurde mit 10 Masse-% eines Polyethylenglycols mit einer mittleren Molekülmasse von 300 als polarem Weichmacher modifiziert. Durch Einbringen von Teilchen einer Methylcellulose einer mittleren Teilchengröße von etwa 70 μm in einer Menge von 7 Masse-% wurde eine entsprechende räumlich vernetzte Struktur auf gebaut, wobei das Zahlenverhältnis der Teilchen des Hämostatikums zu den Teilchen des Adhäsionsmittels etwa 1:1,5 betrug.

In die Struktur des hämostatischen Materials wurde ferner 1 Masse-% Methylparaben als antimikrobielles Mittel eingebracht.

Beispiel 10

Aus faserförmigen Teilchen auf der Basis von Alginsäure und Calciumalginat (1:1) mit einer mittleren Teilchengröße (Länge) von 300 bis 800 μm, die in einer Menge von 76 Masse-% eingesetzt wurden, wurden hämostatische Zäpfchen für stomatologische Applikationszwecke hergestellt. Das Hämostatikum wurde nach Modifizierung mit 8 Masse-% Polyethylenglycol mit Teilchen von Hydroxyethylcellulose einer mittleren Teilchengröße von 65 μm, die in einer Menge von 15 Masse-% eingesetzt wurden, miteinander verbunden, wobei das Zahlenverhältnis der Teilchen des Hämostatikums zu den Teilchen des Adhäsionsmittels etwa 1:2 betrug. In die Raumstruktur des hämostatischen Materials wurde ferner 1 Masse-% Sorbinsäure als antimikrobielles Mittel eingebracht.

Beispiel 11

Aus Collagenteilchen in einer Menge von 85 Masse-% und Teilchen von Methylhydroxyethylcellulose als Adhäsionsmittel, das in einer Menge von 15 Masse-% eingesetzt wurde, wurden hämostatische Zäpfchen für stomatologische Zwecke in Form einer vernetzten Raumstruktur hergestellt, wobei das Zahlenverhältnis der Teilchen des Hämostatikums zu den Teilchen des Adhäsionsmittels 1:1,8 betrug. Die Teilchen des Adhäsionsmittels bildeten mit den faserförmigen Teilchen des Hämostatikums eine räumlich vernetzte Struktur, welche die geforderte hämostatische Wirksamkeit und hohes Sorptionsvermögen aufwies.

Beispiel 12

Durch Aufbringen einer Schicht von räumlich vernetzten faserförmigen Teilchen des Calciumsalzes von Carboxycellulose mit 14 % freien Carboxylgruppen, das in einer Menge von 82 Masse-% der Schicht eingesetzt wurde, auf ein Substrat - (Web-bzw. Strickware bzw. Textilverbundstoff auf der Basis von Baumwolle oder eines Baumwolle-Viskose-Gemischs) wurde ein hämostatisches Verbandmaterial hergestellt.

Die faserförmigen Teilchen der Carboxycellulose, die eine mittlere Teilchengröße von 500 μm aufwiesen, wurden mit 10 Masse-% eines Polyethylenglycols mit einer mittleren Molekülmasse von 300 als polarem Weichmacher modifiziert.

Die derart modifizierten Teilchen des Hämostatikums wurden dann mit Teilchen von Methylhydroxyethylcellulose einer mittleren Teilchengröße von etwa 50 μm, die in einer Menge von 12 Masse-% eingestzt wurden, als Adhäsionsmittel zu einer räumlich vernetzten Struktur verbunden, wobei das Zahlenverhältnis der Teilchen des Hämostatikums zu den Teilchen des Adhäsionsmittels etwa 1:1,5 betrug.

Zur Erhöhung des Sorptionsvermögens der hämostatischen Schicht wurden ferner in die räumlich vernetzte Struktur hydrophile Teilchen von Carboxymethylstärke einer Teilchengröße von 50 bis 100 μm in einer Menge von 16 Masse-% gleichmäßig eingebracht, worauf die erhaltene hämostatische Schicht dann mit einer Gazeunterlage mit Hilfe der Teilchen der Methylhydroxyethylcellulose als Adhäsionsmittel verbunden wurde.

Beispiel 13

Aus faserförmigen Teilchen einer Carboxycellulose mit 18 % freien Carboxylgruppen und einer mittleren Teilchengröße (Länge) im Bereich von 100 bis 600 μm wurde ein Verbandmaterial mit lokalhämostatischer Wirkung durch Aufbringen einer Schicht eines räumlich vernetzten hämostatischen Materials auf eine Baumwolltextilunterlage hergestellt und darauf mittels des Adhäsivums fixiert. Die Carboxycellulose wurde in einer Menge von 75 Masse-% eingesetzt. Durch Zusatz von Teilchen einer Hydroxyethylcellulose einer mittleren Teilchengröße von 50 μm in einer Menge von 15 Masse-% wurde eine räumlich vernetzte Struktur aufgebaut, wobei das Zahlenverhältnis der Teilchen des Hämostatikums zu den Teilchen des Adhäsionsmittels etwa 1:2,5 betrug. Das Sorptionsvermögen des erhaltenen hämostatischen Materials für Blut und Körperflüssigkeiten wurde durch in der Matrix homogen verteilte Carboxymethylstärke in einer Menge von 6 Masse-% und Hydroxyethylstärke in einer Menge von 4 Masse-% erhöht.

Beispiel 14

Aus faserförmigen Teilchen auf der Basis von Alginsäure und Calciumalginat (Verhältnis etwa 1:1) wurde durch Aufbringen einer Schicht des hämostatischen Materials auf eine Baumwollgazeunterlage ein hämostatisches Verbandmaterial hergestellt. Hierzu wurden die faserförmigen Teilchen des Hämostatikums, das eine Teilchengröße von 300 bis 800 μm besaß und in einer Menge von 82 Masse-% eingesetzt wurde, mit Hilfe von Teilchen von Methylcellulose einer mittleren Teilchengröße von 75 μm als Adhäsionsmittel miteinander verbunden, das in einer Menge von 12 Masse-%, bezogen auf die hämostatische Schicht, eingesetzt wurde. Das Zahlenverhältnis der Teilchen des Hämostatikums zu den Teilchen des Adhäsionsmittels betrug etwa 1:1,5. Durch das Adhäsionsmittel wurden die faserförmigen Teilchen des Hämostatikums zu einer räumlich vernetzten Struktur miteinander verbunden. Die Sorptionskapazität der Schicht wurde durch Zusatz von hydrophiler Hydroxyethylstärke in einer Menge von 6 Masse-% erhöht, wobei die Teilchen der Hydroxyethylstärke gleichmäßig in der hämostatischen Schicht des Verbandmaterials verteilt waren.

Beispiel 15

Durch Aufbringen einer hämostatischen Schicht eines hämostatischen Materials mit vernetzter Raumstruktur auf eine Textilbahn wurde ein Verbandmaterial mit hämostatischer Wirkung hergestellt, wobei die beiden Komponenten mit einem Bindemittel miteinander verbunden wurden. Die hämostatische Schicht bestand aus 84,6 Masse-% faserförmiger Teilchen von Collagen, die mit 15 Masse-% Teilchen von Methylhydroxyethylcellulose einer mittleren Teilchengröße von 50 μm als Adhäsionsmittel zu einer vernetzten Raumstruktur miteinander verbunden wurden, wobei das Zahlenverhältnis der Teilchen des Hämostatikums zu den Teilchen des Adhäsionsmittels etwa 1:2 betrug. Die hämostatische Schicht enthielt ferner 0,4 Masse-% Cinchocain als Lokalanästhetikum.

Beispiel 16

Aus faserförmigen Teilchen des Calciumsalzes von Carboxycellulose mit 14 % freien Carboxylgruppen und faserförmigen Teilchen von Calciumalginat (Massenverhältnis 1:1,5) wurde ein hämostatisches Material mit räumlich vernetzter Struktur hergestellt. Hierzu wurden die Teilchen des Hämostatikums, das eine mittlere Teilchengröße von 500 μm besaß und in einer Menge von 75 Masse-% eingesetzt wurde, mit Hilfe von Teilchen von Methylhydroxyethylcellulose einer mittleren Teilchengröße von 50 μm, die in einer Menge von 12 Masse-% eingesetzt wurden, miteinander zu einer vernetzten Raumstruktur verbunden. Durch Modifizierung mit 15 Masse-% Glycerin wurde die Biegsamkeit des faserförmigen Hämostatikums erhöht.

Beispiel 17

Aus faserförmigen Teilchen auf der Basis von Alginsäure, die zu 30 % in das entsprechende Natriumsalz umgewandelt war und eine mittlere Teilchengröße von 600 μm besaßen und in einer Menge von 84,5 Masse-% eingesetzt wurden, wurde ein hämostatisches Material hergestellt. Hierzu wurden die faserförmigen Teilchen des Hämostatikums mit Hilfe von Teilchen von Hydroxyethylcellulose einer mittleren Teilchengröße von 70 μm, die in einer Menge von 15 Masse-% eingesetzt wurden, zu einer vernetzten Raumstruktur miteinander verbunden. Das hämostatische Material enthielt ferner 0,2 Masse-% an die vorliegenden Carboxylgruppen gebundenes Carbethoxypentadecylammoniumbromid als Bakterizid und 0,3 Masse-% Benzocain als Lokalhämostatikum.

Ansprüche

1. Hämostatisches Material

auf der Basis faserförmiger Teilchen mindestens einer im Kontakt mit Blut hämostatisch wirkenden Substanz als Hämostatikum,

**dadurch gekennzeichnet,**

daß die faserförmigen Teilchen des Hämostatikums mit Teilchen eines Adhäsionsmittels zu einer räumlich vernetzten Struktur gebunden sind.

2. Hämostatisches Material nach Anspruch 1, gekennzeichnet durch Carboxycellulose(n) und/oder deren Alkali-und/oder Calciumsalze, mikrokristallines Collagen und/oder Alginsäure und/oder deren Alkali-und/oder Calciumsalze als Hämostatika.

3. Hämostatisches Material nach Anspruch 1 oder 2, gekennzeichnet durch Methylcellulose, Hydroxyethylcellulose, Hydroxypropylcellulose, Methylhydroxyethylcellulose und/oder Methylhydroxypropylcellulose als Adhäsionsmittel.

4. Hämostatisches Material nach einem der Ansprüche 1 bis 3, gekennzeichnet durch ein Zahlenverhältnis von 1 bis 10 Teilchen des Adhäsionsmittels pro Teilchen des Hämostatikums.

5. Hämostatisches Material nach einem der Ansprüche 1 bis 4, gekennzeichnet durch mindestens 1 Masse-% Adhäsionsmittel, bezogen auf die Gesamtmasse von Hämostatikum und Adhäsionsmittel.

6. Hämostatisches Material nach einem der Ansprüche 1 bis 5, gekennzeichnet durch Carboxymethylstärke und/oder Hydroxyethylstärke als Additive.

7. Hämostatisches Material nach einem der Ansprüche 1 bis 6, dadurch gekennzeichnet, daß die Teilchen des Hämostatikums mit einem polaren Weichmacher imprägniert sind.

8. Hämostatisches Material nach Anspruch 7, dadurch gekennzeichnet, daß die Teilchen des Hämostatikums mit 5 bis 50 Masse-% Weichmacher imprägniert sind.

9. Hämostatisches Material nach einem der Ansprüche 1 bis 8, dadurch gekennzeichnet, daß das Hämostatikum auf der Basis einer Carboxycellulose 8 bis 24 % Carboxylgruppen aufweist, die ggfs. teilweise in Salzform vorliegen.

10. Hämostatisches Material nach einem der Ansprüche 1 bis 9, dadurch gekennzeichnet, daß das Adhäsionsmittel auf der Basis von Methylcellulose, Hydroxyethylcellulose, Hydroxypropylcellulose, Methylhydroxyethylcellulose, und/oder Methylhydroxypropylcellulose einen mittleren Substitutionsgrad von 0,5 bis 1,5 aufweist.

11. Hämostatisches Material nach einem der Ansprüche 8 bis 10, gekennzeichnet durch ein Polyethylenglycol mit einer mittleren Molekülmasse von 150 bis 600 als polaren Weichmacher.

12. Hämostatisches Material nach einem der Ansprüche 8 bis 11, gekennzeichnet durch Glycerin als polaren Weichmacher.

13. Hämostatisches Material nach einem der Ansprüche 1 bis 11, dadurch gekennzeichnet, daß es in seiner räumlich vernetzten Struktur einen Emulgator in einer Menge von 0,1 bis 5 Masse-% verteilt enthält.

14. Hämostatisches Material nach Anspruch 13, gekennzeichnet durch ein Monoglycerid einer $C_{12-18}$-Alkancarbonsäure als Emulgator.

15. Hämostatisches Material nach Anspruch 13 oder 14, gekennzeichnet durch Palmitinsäure- und/oder Stearinsäuremonoglycerid als Emulgatoren.

16. Hämostatisches Material nach einem der Ansprüche 13 bis 15, gekennzeichnet durch ethoxyliertes Sorbitanoleat als Emulgator.

17. Hämostatisches Material nach einem der Ansprüche 1 bis 16, gekennzeichnet durch ein in der räumlich vernetzten Struktur verteiltes Lokalanästhetikum.

18. Hämostatisches Material nach Anspruch 17, gekennzeichnet durch einen Gehalt von bis zu 1 Masse-% an Lokal anästhetikum.

19. Hämostatisches Material nach Anspruch 17 oder 18, gekennzeichnet durch Cinchocainiumchlorid als Lokalanästhetikum.

20. Hämostatisches Material nach einem der Ansprüche 17 bis 19, gekennzeichnet durch Benzocainiumchlorid als Lokalanästhetikum.

21. Hämostatisches Material nach einem der Ansprüche 17 bis 20, gekennzeichnet durch Mesocain und/oder Lidocain als Lokalanästhetika.

22. Hämostatisches Material nach einem der Ansprüche 1 bis 21, gekennzeichnet durch ein in seiner räumlich vernetzten Struktur verteiltes antibakterielles Mittel.

23. Hämostatisches Material nach Anspruch 22, gekennzeichnet durch einen Gehalt von bis zu 5 Masse-% an antibakteriellem Mittel.

24. Hämostatisches Material nach Anspruch 22 oder 23, gekennzeichnet durch Methylparaben als antibakterielles Mittel.

25. Hämostatisches Material nach einem der Ansprüche 22 bis 24, gekennzeichnet durch eine Alkalisalz von Sorbinsäure als antibakterielles Mittel.

26. Hämostatisches Material nach einem der Ansprüche 22 bis 25, dadurch gekennzeichnet, daß in seiner räumlich vernetzten Struktur ein antibakterielles Mittel vom kationoiden Typ an freie Carboxylgruppen der Carboxy cellulose chemisch gebunden ist.

27. Hämostatisches Material nach Anspruch 26, gekennzeichnet durch Cetyltrimethylammoniumbromid als antibakterielles Mittel vom kationoiden Typ.

28. Hämostatisches Material nach Anspruch 26 oder 27, gekennzeichnet durch Carbethoxypentadecyltrimethylammoniumbrommid als antibakterielles Mittel vom kationoiden Typ.

29. Hämostatisches Material nach einem der Ansprüche 1 bis 28, gekennzeichnet durch faserförmige Teilchen des Hämostatikums einer Teilchengröße/Länge im Bereich von 30 bis 800 μm.

30. Hämostatisches Material nach einem der Ansprüche 1 bis 29, gekennzeichnet durch ein Adhäsionsmittel einer mittleren Teilchengröße von 40 bis 70 μm.

31. Verfahren zur Herstellung des hämostatischen Materials nach einem der Ansprüche 1 bis 30,

gekennzeichnet durch

gleichmäßiges Dispergieren oder Einmischen eines Adhäsionsmittels in einem Hämostatikum.

32. Verfahren nach Anspruch 31, gekennzeichnet durch Imprägnieren des Hämostatikums mit oder Dispergieren in einem polaren Weichmacher vor dem Einbringen des Adhäsionsmittels.

33. Verfahren nach Anspruch 31 oder 32, gekennzeichnet durch Verwendung von Carboxycellulose(n) und/oder deren Alkali-und/oder Calciumsalzen, mikrokristallinem Collagen und/oder Alginsäure und/oder deren Alkali-und/ oder Calciumsalzen als Hämostatika.

34. Verfahren nach einem der Ansprüche 31 bis 33, gekennzeichnet durch Verwendung von Methylcellulose, Hydroxyethylcellulose, Hydroxypropylcellulose, Methylhydroxyethylcellulose und/oder Methylhydroxypropylcellulose als Adhäsionsmittel.

35. Verfahren nach einem der Ansprüche 31 bis 34, gekennzeichnet durch Einsatz des Adhäsionsmittels in einem Zahlenverhältnis von 1 bis 10 Teilchen pro Teilchen des Hämostatikums.

36. Verfahren nach einem der Ansprüche 31 bis 35, gekennzeichnet durch Einsatz des Adhäsionsmittels in einer Menge von mindestens 1 Masse-%, bezogen auf die Gesamtmasse von Hämostatikum und Adhäsionsmittel.

37. Verfahren nach einem der Ansprüche 31 bis 36, gekennzeichnet durch Zusatz von Carboxylmethylstärke und/oder Hydroxyethylstärke als Additive.

38. Verfahren nach einem der Ansprüche 32 bis 37, gekennzeichnet durch Einsatz des Weichmachers in einer Menge von 5 bis 50 Masse-%.

39. Verfahren nach einem der Ansprüche 31 bis 38, gekennzeichnet durch Einsatz einer Carboxylcellulose mit 8 bis 24 % Carboxylgruppen, die ggfs. teilweise in Salzform vorliegen, als Hämostatikum.

40. Verfahren nach einem der Ansprüche 31 bis 39, gekennzeichnet durch Einsatz des Adhäsionsmittels auf der Basis von Methylcellulose, Hydroxyethylcellulose, Hydroxypropylcellulose, Methylhydroxyethylcellulose und/oder Methylhydroxypropylcellulose mit einem mittleren Substitutionsgrad von 0,5 bis 1,5.

41. Verfahren nach einem der Ansprüche 32 bis 40, gekennzeichnet durch Verwendung eines Polyethylenglycols mit einer Molmasse von 150 bis 600 oder von Glycerin als Weichmacher.

42. Verfahren nach einem der Ansprüche 31 bis 41, gekennzeichnet durch Verwendung eines Emulgators zum Dispergieren bzw. Einmischen des Adhäsionsmittels in einer Menge von 0,1 bis 5,0 Masse-%.

43. Verfahren nach Anspruch 42, gekennzeichnet durch Verwendung von Monoglyceriden von $C_{12-18}$-Alkancarbonsäuren, Palmitinsäure-und/oder Stearinsäuremonoglycerid und/oder ethoxyliertem Sorbitanoleat als Emulgatoren.

44. Verfahren nach einem der Ansprüche 31 bis 43, gekennzeichnet durch Dispergieren eines Lokalanästhetikums in der räumlich vernetzten Struktur des hämostatischen Materials oder Beschichten des hämostatischen Materials mit einem Lokalanästhetikum.

45. Verfahren nach Anspruch 44, gekennzeichnet durch Verwendung von Cinchocainiumchlorid, Benzocainchlorid, Mesocain und/oder Lidocain als Lokalanästhetika.

46. Verfahren nach Anspruch 45, gekennzeichnet durch Einsatz des Lokalanästhetikums in einer Menge von bis zu 1 Masse-%.

47. Verfahren nach einem der Ansprüche 31 bis 46, gekennzeichnet durch Dispergieren eines antibakteriellen Mittels in der räumlich vernetzten Struktur des hämostatischen Materials oder Aufbringen eines antibakteriellen Mittels auf das hämostatische Material.

48. Verfahren nach Anspruch 47, gekennzeichnet durch Einsatz des antibakteriellen Mittels in einer Menge von bis zu 5 Masse-%.

49. Verfahren nach Anspruch 47 oder 48, gekennzeichnet durch Verwendung von Methylparaben, einem Alkalisalz von Sorbinsäure, Cetyltrimethylammoniumbromid und/oder Carbethoxypentadecyltrimethylammoniumbromid als antimikrobielle Mittel.

50. Verfahren nach einem der Ansprüche 31 bis 49, gekennzeichnet durch Verwendung von faserförmigen Teilchen des Hämostatikums einer mittleren Teilchengröße/Länge im Bereich von 30 bis 800 μm.

51. Verfahren nach einem der Ansprüche 31 bis 50, gekennzeichnet durch Verwendung eines Adhäsionsmittels mit einer mittleren Teilchengröße von 40 bis 70 μm.

52. Formkörper auf der Basis faserförmiger Teilchen eines Hämostatikums, gekennzeichnet durch ein hämostatisches Material nach einem der Ansprüche 1 bis 30.

53. Formkörper nach Anspruch 54, dadurch gekennzeichnet, daß sie in Form von Zäpfchen oder Flachmaterialien vorliegen.

54. Formkörper nach Anspruch 52 oder 53, dadurch gekenn zeichnet, daß sie einen Träger aus einem Textilmaterial oder einem Faservliesmaterial aufweisen, und das hämostatische Material durch das Adhäsionsmittel an den Träger gebunden ist.